# EUROPÄISCHE PATENTSCHRIFT

(11) **EP 1 113 787 B1**
(45) Veröffentlichungstag und Bekanntmachung des Hinweises auf die Patenterteilung: **03.03.2004**
(21) Anmeldenummer: 99948770.5
(22) Anmeldetag: 17.09.1999
(51) Int. Cl.: A61K 9/16

(54) **AGITATIONSUNABHÄNGIGE PHARMAZEUTISCHE MULTIPLE-UNIT-RETARDZUBEREITUNGEN UND VERFAHREN ZU IHRER HERSTELLUNG**
MULTIPLE UNIT CONTROLLED FOOD EFFECT-INDEPENDENT RELEASE PHARMACEUTICAL PREPARATIONS AND METHOD FOR PREPARING THE SAME
PREPARATIONS PHARMACEUTIQUES A LIBERATION CONTROLEE EN FONCTION DE L'AGITATION ET LEUR PROCEDE DE PRODUCTION

(30) Priorität: 18.09.1998 DE 19842753
(43) Veröffentlichungstag der Anmeldung: 11.07.2001
(73) Patentinhaber: Bayer HealthCare AG, 51368 Leverkusen (DE)
(72) Erfinder: KANIKANTI, Venkata-Rangarao, D-51381 Leverkusen (DE); RUPP, Roland, D-51467 Bergisch Gladbach (DE); BRENDEL, Erich, D-42657 Solingen (DE); WEISEMANN, Claus, Apex, NC 27502 (US); CHANTRAINE, Ernst, D-51467 Bergisch Gladbach (DE)
(86) Internationale Anmeldenummer: PCT/EP1999/006882
(87) Internationale Veröffentlichungsnummer: WO 2000/016748

(56) Entgegenhaltungen:
- EP-A- 0 205 282
- ACQUIER, R., ET AL.: "Hydroxypropyl cellulose et liberation des principes actifs I. Influence de la masse moleculaire du polymere et de sa concentration" S.T.P PHARMA SCIENCES, Bd. 2, Nr. 6, 1992, Seiten 469-474, XP000865524
- ACQUIER, R.; ET AL.: "Approche du comportement des hydroxypropylcelluloses en presence d'eau, en fonction de la masse moleculaire et de la concentration" PHARMACEUTICA ACTA HELVETIAE, Bd. 67, Nr. 11, 1992, Seiten 315-320, XP000863695 Bern, CH

## Beschreibung

Die vorliegende Erfindung betrifft oral applizierbare Multiple-Unit-Retarddosisformen in Form einer Kapsel mit kontrollierter und Agitations-unabhängiger Freisetzung und Verfahren zu ihrer Herstellung unter Verwendung eines ausgewählten erodierbaren hydrophilen Polymers.

Für viele Arzneimittel ist es wünschenswert, dass sie nach einmal täglicher Verabreichung eine kontrollierte, langanhaltende und gleichmäßige Freisetzung des Wirkstoffs gewährleisten. Auf diese Weise kann die gewünschte Plasmakonzentration ohne große Schwankungen über einen längeren Zeitraum aufrecht erhalten werden und somit die Arzneimittelsicherheit und die Patienten-Compliance erhöht werden. Formulierungen, die den Wirkstoff auf diese Weise über einen definierten Zeitraum freisetzen, werden als Retardformulierungen bezeichnet. Es sind bereits verschiedene Techniken zu ihrer Herstellung bekannt.

Sehr häufig werden für diesen Zweck Single-Unit-Matrixtabletten eingesetzt, die den Wirkstoff in einer Matrix aus Polymeren und einigen pharmazeutischen Hilfsstoffen enthalten. Das Polymer kann entweder hydrophil oder hydrophob sein oder eine Mischung daraus darstellen. Mittlerweile sind Matrixtabletten mit hydrophilen Polymeren sehr beliebt geworden, da diese vergleichsweise preiswert, nichttoxisch, auf herkömmlichen Anlagen verarbeitbar sind, usw.

So sind beispielsweise in EP-A-0 205 282 verzögert freisetzende orale pharmazeutische Zusammensetzungen für die Anwendung in der Mundhöhle beschrieben, wobei durch die Verwendung von Hydroxypropylcellulose als hydrophilem Polymer die Adhäsion an der Mundschleimhaut verbessert werden kann.

Eine weitere Methode ist das Ummanteln von Zubereitungsformen mit gepufferten bzw. pH-abhängigen Umhüllungen, die eine kontrollierte Freisetzung in bestimmten Bereichen des Magen-Darm-Traktes gewährleisten soll.

Während die Erosions-Matrixtabletten anfällig sind gegenüber mechanischer Beanspruchung, insbesondere hydrodynamischen Belastungen, sind die pH-gesteuerten Formulierungen anfällig gegenüber pH-Wert-Änderungen im Magen-Darm-Trakt. Während sich die Tablette durch den Magen-Darm-Trakt bewegt, variiert sowohl der pH-Wert, als auch die mechanische Beanspruchung, insbesondere auch in Abhängigkeit von Art und Menge der Füllung des Magens und des Verdauungstraktes. Diese Abhängigkeit der Wirkstofffreisetzung wird als "Agitationsabhängigkeit" oder als "Food-Effekt" bezeichnet. Es zeigt sich, dass die Freisetzungsrate der meisten Retardformulierungen abhängig ist von der Nahrungsaufnahme und somit unterschiedliche Wirkprofile auftreten in Abhängigkeit davon, ob die Einnahme des Arzneimittels vor, während oder nach einer Mahlzeit erfolgt.

Es gibt zahlreiche Versuche die unerwünschte Variabilität dieses "Food-Effekts" auszuschalten bzw. zu minimieren. Für erosionskontrollierte Zubereitungen wurde ein annähernd agitationsunabhängiges Single-Unit-System beschrieben, das jedoch technisch sehr aufwendig und daher inpraktikabel ist (vgl. W.D. Lindner et al. Farm., 51 (1996) 263). Als weitere Möglichkeit einer agitationsunabhängigen Zubereitung wurde ein Single-Unit-osmotisches Pumpsystem beschrieben und teilweise erfolgreich vermarktet. Hierbei wird der Wirkstoff durch definierte Öffnungen oder Poren einer Kammer nach außen gepreßt, wobei der Preßdruck durch ein quellendes Polymer erzeugt wird, dessen Wasseraufnahme osmotisch gesteuert wird (vgl. US-Pat 4 449 983, US-Pat 4 203 400 und US-Pat 4 327 725).

Die Probleme und Nachteile der bisher vorgeschlagenen und eingesetzten agitationsunabhängigen Retardformulierungen sind bekannt und unter anderem in der Beschreibung von EP 0 425 298.A2 dargelegt. Gemäß dieser Anmeldung wird versucht die Agitationsunabhängigkeit von salzbildenden Wirkstoffen durch unterschiedliches Ummanteln mit schwerlöslichen Polymeren zu erreichen. Die Nachteile dieses Verfahrens liegen ebenfalls in den technisch aufwendigen Verfahrensmaßnahmen und in der Tatsache, dass nur bestimmte salzbildende und somit leicht lösliche Wirkstoffe eingesetzt werden können.

Als Multiple-Unit-Formulierung werden solche Formulierungen bezeichnet, die im Gegensatz zu sogenannten Single-Unit-Formulierungen wie Tabletten aus mehreren kleinen Partikeln wie z.B. Pellets, Granulaten, Minitabletten oder Körnern bestehen, die z.B. in einer Kapsel enthalten sind. Im Magen-Darm-Trakt liegen diese Partikel dann unabhängig voneinander vor. Solche Multiple-Unit-Formulierungen weisen eine Reihe von Vorteilen im Vergleich zu Single-Unit-Formulierungen auf. Sie sorgen für eine gleichmäßigere Absorption des Wirkstoffs und für geringere inter- und intraindividuelle Schwankungen der pharmacokinetischen Profile. Weiterhin lassen sich so in einfacher Weise verschiedene Wirkstoffe und Dosierungen z.B. in Kapseln einbringen. Diese Formulierungen können so den unterschiedlichen medizinischen Anforderungen ohne großen Aufwand angepaßt werden.

Die Aufgabe der vorliegenden Erfindung ist darin zu sehen, agitationsunabhängige Multiple-Unit-Retardformulierungen, d.h. Formulierungen ohne störenden Food-Effekt für alle Arten von Wirkstoffen, insbesondere für schwerlösliche Wirkstoffe zur Verfügung zu stellen, die in einfacher Weise hergestellt werden können.

Multiple-Unit-Retardformulierungen gemäß der vorliegenden Erfindung sind Formulierungen, die im USP Paddletest mit Apparat II 80 % des Wirkstoffs innerhalb von 4 bis 14 Stunden, vorzugsweise innerhalb von 6 bis 12 Stünden freisetzen, bezogen auf die gesamte Wirkstoffmenge in der Formulierung.

Agitationsunabhängig gemäß der vorliegenden Erfindung sind Formulierungen, die im USP XXII Paddletest mit 900 ml Freisetzungsmedium, pH 6,8 bei einer Rührgeschwindigkeit von 50 UpM und von 150 UpM eine maximale Freisetzungsdifferenz von ± 10 %, vorzugsweise ± 5 % aufweisen.

Die Rührgeschwindigkeit des Paddletests nach USP wurden ausgewählt im Hinblick auf die Publikation B. Abrahamsson et al., Eur. J. Pharm. Sci., 46 (1998) 69, wonach die mechanische Beanspruchung einer Tablette im Magen-Darm-Trakt etwa mit den Bedingungen zu vergleichen ist, die einer Rührbewegung im Paddletest mit bis ca. 150 U/min entsprechen.

Diese Aufgabe läßt sich erfindungsgemäß dadurch lösen, dass man
a) als hydrophiles Polymer Hydroxypropylcellulose (HPC) mit einem mittleren Molekulargewicht von 250 000 bis 1 200 000, vorzugsweise 350 000 bis 1 150 000 in einer Menge von 40 bis 95 Gew.-%, vorzugsweise 45 bis 90 Gew.-%, bezogen auf das Wirkstoff-Polymergemisch, und einem molaren Substitutionsgrad von ≥3 als retardierendes Erosionsmaterial einsetzt und
b) die Wirkstoff-Polymer-Kombination in kleine Partikel wie Pellets, Granulate oder Minitabletten mit einem maximalen Durchmesser von 0,2 bis 3,0 mm, vorzugsweise von 0,5 bis 2 mm, überführt und
c) diese dann in einer wirksamen Dosierung in Kapseln füllt.

Es kann auch HPC mit einem mittleren Molekulargewicht von 700 000 bis 1 200 000, vorzugsweise 850 000 bis 1 150 000 eingesetzt werden.

Unter maximalem Durchmesser wird hierbei die größte Längenausdehnung des Partikels verstanden; sie liegt erfindungsgemäß bei 0,2 bis 3 mm.

Gewünschtenfalls können die Minipartikel lackiert werden und auch weitere übliche pharmazeutische Hilfsstoffe hinzufügt werden.

Diese retardierten Minipartikel können in einfacher Weise in den gewünschten Dosiseinheiten als Multiple-Unit-Retarddosisformen hergestellt und verabreicht werden, wie z.B. in Hardgelatinekapseln.

Bei Kenntnis des Standes der Technik war es nicht naheliegend, dass durch die Auswahl des oben genannten erodierbaren hydrophilen Polymers HPC und gleichzeitiger Limitierung der Maximalgröße der Wirkstoff-enthaltenden Polymerpartikel auf höchstens 3 mm Durchmesser eine agitationsunabhängige Retardformulierung hergestellt werden kann. Es war vielmehr zu erwarten, dass gerade leicht erodierbare hydrophile Polymere einen besonders starken Agitationseffekt bzw. Foodeffekt zeigen würden. Es ist z.B. bekannt, dass Nifedipin-enthaltende Matrixtabletten mit Durchmessern von 9 oder 10 mm, die HPC oder HPMC (Hydroxypropylmethylcellulose) als hydrophiles Polymer enthalten, stark agitationsabhängig sind und einen starken Food-Effekt zeigen. (Vgl. Adalat CC®; EP 0 299 211 und B. Abrahamsson et al, J. Controlled Rel., 52 (1998) 301).

Andererseits sind auch Minitabletten zur oralen Anwendung seit einiger Zeit bekannt und beschrieben (vgl. Colombo et al., Acta Technol. Legis. med. 1992, 3 (3), 137). Es ist aber bisher nicht bekannt, dass die erfindungsgemäßen Partikel mit einem maximalem Durchmesser von 3 mm agitationsunabhängige Retardformulierungen darstellen.

Zur Lösung der erfindungsgemäßen Aufgabe ist die Kombination der Elemente a), b) und c) erforderlich. Eigene Versuche mit Minierosionstabletten, welche als Wirkstoff Nifedipin enthalten, die zwar einem Durchmesser von 2 mm besitzen, aber als erodierbares Polymer eine Mischung aus Hydroxyethylcellulose (HEC) und Hydroxyproylmethylcellulose (HPMC) enthalten, zeigen eine signifikante Agitationsabhängigkeit.

Überraschenderweise wurde gefunden, dass durch die Kombination der Auswahl des erodierbaren hydrophilen Polymers HPC und die Reduzierung der Größe der einzusetzenden Minipartikel auf maximal 3 mm Durchmesser in einfacher und effektiver Weise agitationsunabhängige Multiple-Unit-Retardformulierungen erhalten werden können.

Nach orientierenden Tests zeigten die erfindungsgemäßen Formulierungen praktisch keine Nahrungsmittelabhängigkeit.

Die Herstellung der erfindungsgemäß einzusetzenden Pellets, Granulate, Minitabletten oder Körner erfolgt nach üblichen Methoden. Neben den herkömmlichen Formulierungsmethoden, in denen HPC mit dem Wirkstoff und gegebenenfalls weiteren Hilfsstoffen unter Verwendung von Wasser oder organischen Lösungsmitteln granuliert wird, kann auch die Verwendung von Schmelzextrusionsmethoden in vorteilhafter Weise eingesetzt werden. Solche Schmelzextrusionsmethoden sind seit langem bekannt. Variationen dieser Schmelzextrusion werden auch in der jüngeren Patentliteratur vorgeschlagen (vgl. DE 195 04 831.8, EP 240 904, US-PS 5 456 923, EP 544 144 und insbesondere WO 96/25149).

Viele der bisher bekannten Methoden der Schmelzextrusion weisen gegenüber den erfindungsgemäß einsetzbaren Methoden eine Reihe von Nachteilen auf. So werden zur Herstellung eines Extrudates häufig mindestens zwei Polymere, z.B. ein wasserlösliches und ein wasserunlösliches verwendet. Durch die Notwendigkeit von zusätzlichen Weichmachern oder anderen Hilfsstoffen kann das Verhältnis Hilfsstoff/Arzneistoff ungünstig beeinflußt werden, so dass das fertige Produkt sehr voluminös und auch teuer ist. Gemäß der vorliegenden Erfindung können die agitationsund nahrungsunabhängigen Formulierungen bereits durch einfaches Mischen und Extrudieren des gewünschten Wirkstoffs mit HPC erhalten werden.

Gewünschtenfalls können natürlich auch für die erfindungsgemäßen Multiple-Unit-Retard-Formulierungen weitere Hilfsstoffe eingesetzt werden wie z.B. Magnesiumstearat oder Filmüberzüge oder Lackierungen, die das Aneinanderkleben der Partikel verhindern. Diese Hilfsstoffe haben jedoch keinen direkten Einfluß auf die agitationsunabhängige oder keinen Food-Effekt aufweisende Retardwirkung der erfindungsgemäßen Zubereitung.

Es ist auch möglich, für die Herstellung der Minitabletten neben dem wesentlichen Polymer HPC weitere hydrophile und wasserunlösliche Polymere wie z:B. Polymethacrylatester einzusetzen. Ein Beispiel ist das bekannte Ammonio Methacrylat Copolymer Typ B (Eudragit® RS PO).

Gegenstand der vorliegenden Erfindung sind auch Verfahren zur Herstellung von Multiple-Unit-Retarddosisformen, dadurch gekennzeichnet, dass mindestens ein therapeutisch wirksamer Stoff und HPC mit einerm mittleren MG von 250 000 bis 1 200 000 als hydrophiles thermoplastisches, aber pharmazeutisch unbedenkliches Polymer sowie gegebenenfalls weitere übliche pharmazeutische Hilfsstoffe, die jedoch nicht zum Retardeffekt beitragen, gemischt, granuliert und tablettiert oder gemischt, extrudiert und granuliert werden zu Partikeln mit einem maximalen Teilchendurchmesser von 3 mm und diese dann in eine geeignete orale Applikationsform überführt werden.

Beim Extrusionsverfahren werden therapeutisch wirksame Arzneistoffe und das Polymer entweder gleichzeitig, ohne vorheriges Mischen, oder als Mischung, nach vorherigem Mischen, in einem normalen Extruder, bevorzugterweise einem Doppelschneckenextruder, gefördert, welcher vorher auf eine Temperatur erhitzt wurde, bei der das Polymer und der Arzneistoff nicht abgebaut werden. Hierbei beträgt der Temperaturbereich an der Austrittsdüse des Extruders 50 bis 220°C, vorzugsweise 80 bis 210°C und besonders 100-180°C. Im Bereich des Produkteintritts in den Extruder beträgt die Temperatur um 25°C. Die Temperatur im Zwischenbereich des Extruders liegt zwischen der Temperatur im Produkteintrittsbereich des Extruders und der Temperatur an der Austrittsdüse des Extruders.

Die homogene Mischung erweicht während des Durchlaufs durch den Extruder und wird am Ende durch eine Platte, die mindestens eine Düse mit einem definierten Durchmesser von ca. 0,2 bis 3,0 mm, vorzugsweise von 0,5 bis 2,0 mm enthält, gepreßt. Die extrudierten Stränge, die beim Austritt aus der Extruderdüse noch weich sind und bei Raumtemperatur schnell fest werden, werden unmittelbar nach ihrem Austritt zu Granulat mit einem Teilchendurchmesser von ca. 0,2 bis 3 mm, vorzugsweise 0,5 bis 2 mm, geschnitten. Alternativ werden die extrudierten Stränge sofort (on-line) granuliert (z.B. Wasser-Ring-Granulation oder Unterwasser-Granulation oder Luft-Granulation) oder sofort in Stücke geschnitten. Bevorzugt ist die Luftgranulation. Die erhaltenen Extrudate können direkt in Hartgelatinekapseln gefüllt werden. Als besondere Ausführungsform hat es sich als vorteilhaft erwiesen, die erhaltenen Extrudate vor ihrer Einfüllung in Gelatinekapseln noch zu lackieren, vorzugsweise mit einem wasserunlöslichen aber wasserdurchlässigen und nicht gelbildenden Polymer.

Diese Retarddosisform gemäß der vorliegenden Erfindung ist nicht anfällig gegenüber mechanischer Beanspruchung bzw. hydrodynamischer Belastung im Magen-Darm-Trakt; die Rate der Wirkstofffreisetzung hängt daher nicht von der mechanischen Beanspruchung und der hydrodynamischen Belastung ab, der das Produkt ausgesetzt ist und ist unabhängig vom Füllungsgrad des Magens. Die Retarddosisform weist also keinen Food-Effekt auf.

Die Formulierung gemäß der vorliegenden Erfindung kann auch mit bekannten Tablettierungsprozessen hergestellt werden, bei denen die Inhaltsstoffe z.B. in bekannter Weise granuliert, gleitfähig gemacht und zu Mikrotabletten von einem Durchmesser ≤3 mm, vorzugsweise ≤2 mm komprimiert werden.

Die agitationsunabhängige Retardierung wird, wie bereits erwähnt, im Gegensatz zu den Formulierungen nach dem Stand der Technik bei der vorliegenden Erfindung durch die Kombination a) des verwendeten Polymers HPC und b) den maximalen Durchmesser erreicht, während die Beschichtung lediglich dazu dient, die Dosisform vor dem Verkleben zu schützen.

Bei dem zu verwendenden Wirkstoff kann es sich um beliebige oral zu verabreichende Arzneistoffe handeln, wie z.B. Antiinfektiva, Kreislaufmittel, Antimykotika, Antidepressiva, Antidementika, Antiepileptika, Antiphlogistika, Analgetika, Antiasthmatika, Antithrombotika Antitumormittel, Antimalariamittel, nichtsteroidale entzündungshemmende Mittel (NSAID), Diuretika, Antiarrhythmica, blutzuckersenkende Mittel, ACE-Hemmer, Sedativa, Decongestiva, Antihistaminika oder Lipidsenker. Lipidsenker können unter anderem Apo B-Inhibitoren oder MTP-Inhibitoren sein. Von besonderem Interesse sind die Apo B-Inhibitoren gemäß EP 705 831, auf die hier ausdrücklich Bezug genommen wird. Von ganz besonderem Interesse ist die Substanz 2-Cyclopentyl-2-[4-(2,4-dimethyl-pyrido[2,3-b]indol-9-ylmethyl)-phenyl]-*N*-(2-hydroxy-1-phenyl-ethyl)acetamid. Für die Zwecke der vorliegenden Erfindung werden nur diejenigen Arzneistoffe eingearbeitet, die sich unter den Temperaturen und Verarbeitungsbedingungen nicht zersetzen. Die zu verabreichende Wirkstoff-menge pro Dosiseinheit kann je nach Art des Arzneistoffs und der Freisetzungsrate innerhalb weiter Grenzen variiert werden. Es hat sich als vorteilhaft erwiesen, auf einen Gew.-Teil Wirkstoff 0,8 bis 10 Gew.-Teile, vorzugsweise 1 bis 5 Gew.-Teile, des gelbildenden Polymers einzusetzen.

Im Gegensatz zu den bisher bekannten Techniken wird zur Retardation gemäß der vorliegenden Erfindung nur ein einziges Polymer benötigt. Die gewünschte Freisetzungsrate erhält man durch Variation der Herstellungsparameter. Die Arzneistofffreisetzungsrate wird z.B. beeinflußt durch die Arzneistoffkonzentration im Endprodukt oder durch Verfahrensparameter der Extrusion, wie die Schneckengeometrie, die Extrusionsrate, die Extrusionstemperatur, der Durchmesser und die Oberfläche des Extrudats, die Viskosität und Molekulargewicht des Polymeren, usw.

Wie bereits erwähnt können auch weitere übliche Hilfsstoffe verwendet werden, die bei der Herstellung von festen Dosisformen in der Pharmazie üblich und aus der Literatur bekannt sind. Keiner dieser Hilfsstoffe ist jedoch notwendig um die erfindungsgemäß gewünschte Verzögerung der Freisetzung des Arzneistoffs und die Agitationsunabhängigkeit wesentlich zu beeinflussen. Diese Hilfsstoffe dienen vielmehr nur dazu, das Verfahren flexibler zu machen.

Man lackiert die Extrudate oder Minitabletten gegebenenfalls z.B. mit pH-unabhängigen wäßrigen Dispersionen wie einer Ethylcellulose-Dispersion (z.B. Aquacoat EC 30 Trademark of FMC) oder einem Poly(ethylacrylat, -methylmethacrylat) 2:1 (z.B. Eudragit NE 30 D Trademark of Röhm Pharma). Außerdem kann ein Weichmacher wie z.B. Triethylcitrat oder Tween 20 verwendet werden, damit der Lackfilm bei der Lagerung nicht spröde wird. In die Lacksuspension kann zusätzlich Magnesiumstearat als Antiklebmittel eingearbeitet werden. HPMC dient als Porenbildner. Der Lack hat im wesentlichen keinen Einfluß auf die Freisetzungsrate, ausgenommen, dass es während der ersten 1-2 Stunden nach der Verabreichung zu einer Verzögerung des Einsetzens der Freisetzung kommen.kann (Lag-Zeit).

Als typische Lacksuspensionen für Minitabletten und Extrudate seien genannt:
(Alle Angaben in Gew.-%)
A. 30 - 60 % (bevorzugt 40 %) Eudragit® NE 30 D Dispersion; 3 - 10 % (bevorzugt 5 %) HPMC 3 cP; 0,05 - 0,5 % (bevorzugt 0,1 %) Tween 20; 1 - 7,5 % (bevorzugt 2,5 %) Magnesiumstearat und vollentsalztes Wasser bis 100 %.
B. 15 - 30 % (bevorzugt 25 %) Aquacoat® EC 30 D Dispersion; 3 - 10 % (bevorzugt 4 - 5 %) HPMC 15 cP; 0,5 - 4 % (bevorzugt 2 %) Triethylcitrat und vollentsalztes Wasser bis 100 %.

Die Lacksuspensionen z.B. werden hergestellt, indem man zunächst HPMC und den Weichmacher getrennt in Wasser löst und dann mit der Dispersion des Filmbildners mischt. Bei Anwesenheit von Magnesiumstearat wird dieses vor der Zugabe der Eudragit-NE-30-D-Dispersion in der wäßrigen Lösung von HPMC und Weichmacher dispergiert.

Die gegebenenfalls lackierten Partikel der erfindungsgemäßen Wirkstoff-Polymerkombination wie Pellets, Granulate, Minitabletten oder Körner können nach üblichen Methoden in Kapseln gefüllt, zu Tabletten gepreßt oder zu sonstigen bekannten Verabreichungsformen oder Fertigarzneimitteln weiterverarbeitet werden.

Die Erfindung wird anhand der nachfolgenden Beispiele näher erläutert.

### Einfluß der Paddle-Apitation auf die Wirkstoffreisetzung

Die Wirkstofffreisetzung aus den erfindungsgemäßen Beispielen 16 und 17 sowie aus dem Vergleichsbeispiel A über die Zeit wurde in einem USP XXII-Paddle-Test untersucht. Dabei zeigte sich, dass bei den Beispielen 16 und 17 die Wirkstofffreisetzung bei 50 und 150 Umdrehungen pro Minute (UpM) über einen Zeitraum von 14 Stunden (also bis zur vollständigen Freisetzung) ummaximal 5 % auseinanderlag, während beim Vergleichsbeispiel A Freisetzungs-differenzen von bis zu 50 % auftraten.

### Vergleichsbeispiel A

19,4 Gew. Teile Hydroxypropylmethylcellulose (Viskosität 100 000 cP, Typ 2208) und 45,3 Gew.- Teile Hydroxyethylcellulose (Viskosität 15 000 cP) werden mit einer wäßrigen Suspension von Nifedipin (30 Gew.-Teile) und Hydroxypropylcellulose (2 Gew.-Teile) einer Viskosität <10 cP granuliert. Das erhaltene Granulat wird mit Magnesiumstearat gleitfähig gemacht und zu 2 mm Minitabletten von 6,4 mg komprimiert. Die Minitabletten werden auf herkömmliche Weise mit einer wäßrigen Dispersion von Eudragit NE 30 D, Magnesiumstearat, Tween 20®, Hydroxypropylmethylcellulose 3 cP und Wasser lackiert. Pro kg Minitabletten werden 0,6 kg Lacksuspension A aufgesprüht. Einige lackierte Minitabletten mit einem Äquivalent von 30 mg Nifedipin werden verkapselt.

### Ausführungsbeispiele

### Beispiel 1

3 kg des Arzneistoffs Nifedipin werden mit 7 kg hochviskosem HPC (MG 400 000 von Nippon Soda, Japan) gemischt. Die Mischung wird auf einem Doppelschneckenextruder mit zwei Austrittsdüsen mit einem Durchmesser von 2 mm verarbeitet. Das Material wird bei einer Düsentemperatur von 150°C extrudiert. Die Temperatur der verschiedenen Untereinheiten im Extruderzylinder wird auf eine Temperatur eingestellt, die mindestens etwa 10°C unter der Düsentemperatur liegt. Das Extrudat wird in etwa 2 mm lange Zylinder geschnitten und in einer Wirbelschichtlackieranlage lackiert. Pro kg Extrudat werden 0,6 kg der Lacksuspension A aufgesprüht. Die Lackierung erfolgt unter üblichen Bedingungen.

### Beispiel 2

Analog Beispiel 1, jedoch werden 2 kg Nifedipin mit 8 kg des gleichen Polymertyps gemischt.

### Beispiel 3

Analog Beispiel 1, jedoch beträgt die Düsentemperatur 160°C.

### Beispiel 4

Analog Beispiel 1, jedoch beträgt der Düsendurchmesser 1, 4 mm.

### Beispiel 5

Analog Beispiel 1, jedoch beträgt der Düsendurchmesser 0,8 mm.

### Beispiel 6

Analog Beispiel 1, jedoch wurden die extrudierten Stränge zunächst in etwa 3 mm lange Zylinder geschnitten.

### Beispiel 7

Analog Beispiel 1, jedoch wurden die etwa 2 mm langen geschnittenen Zylinder nicht lackiert.

### Beispiel 8

Analog Beispiel 1, jedoch beträgt die Düsentemperatur 140°C.

### Beispiel 9

Analog Beispiel 1, jedoch wird als Polymer HPC mit einem mittleren Molekulargewicht von ca. 850 000 (Fa. Hercules, USA) verwendet.

### Beispiel 10

Analog Beispiel 1, jedoch wird als Polymer HPC mit einem mittleren Molekulargewicht von ca. 1 000 000 (Fa. Hercules, USA) verwendet.

### Beispiel 11

Analog Beispiel 1, jedoch wird als Arzneistoff Nisoldipin verwendet.

### Beispiel 12

Analog Beispiel 1, jedoch wird als Arzneistoff Nimodipin verwendet, HPC (MG 400 000, Nippon Soda, Japan) eingesetzt, und die Düsentemperatur beträgt 110°C.

### Beispiel 13

Die gleiche Zusammensetzung wie in Beispiel 1 wird in einer kommerziell erhältlichen Extrusions- und Granulationsvorrichtung unter den gleichen Extrusionsbedingungen extrudiert und dann durch das Wasser-Ring-Verfahren sofort granuliert und getrocknet. Die entstandenen Extrudate waren leicht gerundet und dadurch besser verarbeitbar.

### Beispiel 14

Die gleiche Zusammensetzung wie in Beispiel 1 wird in einer kommerziell erhältlichen Extrusionsvorrichtung extrudiert mit einer Düsenplatte 40 x 0,8 mm oder 36 x 1,3 mm Bohrungen, und dann durch Luft-Granulation sofort on-line granuliert und getrocknet. Die entstandenen Extrudate waren besser verarbeitbar. Die Granulate wurden wie in Beispiel 1 lackiert.

### Beispiel 15

Die gleiche Zusammensetzung wie in Beispiel 1 wird durch einen Extruder mit Austrittdüsen mit einem Durchmesser von 1 mm extrudiert, der extrudierte Strang durch Besprühen mit Wasser gekühlt und sofort granuliert und getrocknet. Die erhaltenen Extrudate werden wie in Beispiel 1 beschrieben, weiterverarbeitet.

### Beispiel 16

250 Teile (Gew.) Hydroxypropylcellulose (MG 1 000 000; Viskosität 1 500 bis 3 000 cP (1 % w/v; 25°C)) werden mit einer wäßrigen Suspension von Nifedipin (30 Teile) und Hydroxypropylcellulose (2 Teile) einer Viskosität <10 cP granuliert. Das erhaltene Granulat wird mit Magnesiumstearat (1,5 Teile) gleitfähig gemacht und zu 2 mm-Minitabletten von 6,5 mg komprimiert. Die Minitabletten werden auf herkömmliche Weise mit einer wäßrigen Dispersion von Eudragit NE 30 D, Magnesiumstearat, Tween 20®, Hydroxypropylmethylcellulose 3 cP und Wasser lackiert. Pro kg Minitabletten werden 0,6 kg Lacksuspension A aufgesprüht. Einige lackierte Minitabletten mit einem Äquivalent von 30 mg Nifedipin werden verkapselt.

### Beispiel 17

Hydroxypropylcellulose entsprechend Beispiel 16 (42,6 Teile) wird mit Eudragit® RS PO (40,8 Teile) vermischt und mit einer wäßrigen Suspension von Nifedipin (30 Teile) und Hydroxypropylcellulose (2 Teile) einer Viskosität <10 cP granuliert. Das erhaltene Granulat wird mit Magnesiumstearat (1,5 Teile) gleitfähig gemacht und zu 2 mm-Minitabletten von 6,5 mg komprimiert. Die Minitabletten (2 mm Durchmesser) werden analog Beispiel 16 lackiert.

### Beispiel 18

1 kg 2-Cyclopentyl-2-[4-(2,4-dimethyl-pyrido[2,3-b]indol-9-yl-methyl)-phenyl]-*N*-(2-hydroxy-1-phenyl-ethyl)acetamid werden mit 2 kg HPC (MG 250 000-400 000, Nippon Soda, Japan) gemischt. Die Mischung wird auf einem Doppelschneckenextruder mit zwei Austrittdüsen mit einem Durchmesser von 2 mm verarbeitet. Das Material wird bei einer Düsentemperatur von 215°C extrudiert. Das Extrudat wird in etwa 2 mm lange Zylinder geschnitten und in einer Wirbelschichtlackieranlage analog Beispiel 1 lackiert.

Falls nicht ausdrücklich anders angegeben, soll der Begriff "Teile" in der vorliegenden Anmeldung immer als "Gew.-Teile" verstanden werden.

## Patentansprüche

1. Verfahren zur Herstellung einer oral applizierbaren Multiple-Unit-Retarddosisformulierung mit kontrollierter Agitations-unabhängiger Freisetzung, **dadurch gekennzeichnet, dass** man das hydrophile Polymer HPC mit einem mittleren Molekulargewicht von 250 000 bis 1 200 000 in einer Menge von 40 bis 95 Gew.-%, bezogen auf das Wirkstoff-Polymergemisch, und einem molaren Substitutionsgrad von mindestens 3 als retardierendes Erosionsmaterial mit mindestens einem pharmazeutischen Wirkstoff mischt, diese Wirkstoff-Polymermischung in kleine Partikel mit einem Durchmesser von 0,2 bis 3,0 mm überführt und diese dann in einer wirksamen Dosierung in Kapseln füllt.

2. Verfahren zur Herstellung einer Formulierung gemäß Anspruch 1, **dadurch gekennzeichnet, dass** man HPC in einer Menge von 45 bis 90 Gew.-% einsetzt.

3. Verfahren zur Herstellung einer Formulierung gemäß Anspruch 1, **dadurch gekennzeichnet, dass** man HPC mit einem mittleren Molekulargewicht von 350 000 bis 1 150 000 einsetzt.

4. Verfahren zur Herstellung einer Formulierung gemäß Anspruch 1, **dadurch gekennzeichnet, dass** die Partikel einen maximalen Durchmesser von 0,5 bis 2 mm besitzen.

5. Verfahren zur Herstellung einer Formulierung gemäß Anspruch 1, **dadurch gekennzeichnet, dass** man die Partikel durch Schmelzextrusion und Granulation herstellt.

6. Verfahren zur Herstellung einer Formulierung gemäß Anspruch 1, **dadurch gekennzeichnet, dass** man die Partikel vor dem Füllen in Kapseln zusätzlich lackiert.

7. Verfahren zur Herstellung einer Formulierung gemäß Anspruch 1, **dadurch gekennzeichnet, dass** man als Wirkstoff Nifedipin verwendet.

8. Verwendung von Partikeln gemäß Anspruch 1 zur Herstellung von Fertigarzneimitteln in Form von Kapseln.

9. Oral applizierbare Multiple-Unit-Retarddosisformulierung mit kontrollierter Agitations-unabhängiger Freisetzung erhältlich gemäß Anspruch 1.

10. Oral applizierbare Multiple-Unit-Retarddosisformulierung mit kontrollierter Agitations-unabhängiger Freisetzung in Form einer Kapsel, enthaltend kleine Partikel in einer wirksamen Dosierung mit einem Durchmesser von 0,2 bis 3,0 mm, bestehend aus einer Mischung des hydrophilen Polymer HPC mit einem mittleren Molekulargewicht von 250 000 bis 1 200 000 in einer Menge von 40 bis 95 Gew.-%, bezogen auf das Wirkstoff-Polymergemisch, und einem molaren Substitutionsgrad von mindestens 3 und einem pharmazeutischen Wirkstoff.

## Claims

1. Process for the production of an orally administrable multiple-unit sustained-release dose formulation having controlled agitation-independent release, **characterized in that** the hydrophilic polymer HPC having an average molecular weight of 250 000 to 1 200 000 is mixed in an amount from 40 to 95% by weight, based on the active compound-polymer mixture and a molar degree of substitution of at least 3, as a release-sustaining erosion material, with at least one pharmaceutical active compound, this active compound-polymer mixture is converted into small particles having a diameter of 0.2 to 3.0 mm and these are then filled into capsules in an efficacious dosage.

2. Process for the production of a formulation according to Claim 1, **characterized in that** HPC is employed in an amount from 45 to 90% by weight.

3. Process for the production of a formulation according to Claim 1, **characterized in that** HPC having an average molecular weight of 350 000 to 1 150 000 is employed.

4. Process for the production of a formulation according to Claim 1, **characterized in that** the particles have a maximum diameter of 0.5 to 2 mm.

5. Process for the production of formulations according to Claim 1, **characterized in that** the particles are produced by melt extrusion and granulation.

6. Process for the production of a formulation according to Claim 1, **characterized in that** the particles are additionally lacquered before being filled into capsules.

7. Process for the production of a formulation according to Claim 1, **characterized in that** the active compound used is nifedipin.

8. Use of particles according to Claim 1 for the production of finished medicaments in the form of capsules.

9. Orally administrable multiple-unit sustained-release dose formulations having controlled agitation-independent release obtainable according to Claim 1.

10. Orally administrable multiple-unit sustained-release dose formulation having controlled agitation-independent release in the form of a capsule containing small particles in an efficacious dose with a diameter of 0.2 to 3.0 mm consisting of a mixture of the hydrophilic polymer HPC having an average molecular weight of 250 000 to 1 200 000 in an amount of 40 to 95% by weight, based on the active compound-polymer mixture, and a molar degree of substitution of at least 3 with a pharmaceutical action compound.

## Revendications

1. Procédé de préparation d'une formulation à dose retard de type multiple unit, avec libération contrôlée en fonction de l'agitation, administrable oralement, **caractérisé en ce que** l'on mélange le polymère hydrophile HPC avec un poids moléculaire moyen allant de 250 000 à 1 200 000 en une quantité allant de 40 à 95% en poids, sur base du mélange agent actif-polymère, et un taux de substitution molaire d'au moins 3, en tant que matériau d'érosion à effet retard, avec au moins un agent actif pharmaceutique, ce mélange agent actif-polymère est converti en petites particules ayant un diamètre de 0,2 à 3,0 mm et celles-ci sont alors disposées dans une capsule en un dosage actif.

2. Procédé de préparation d'une formulation suivant la revendication 1, .**caractérisé en ce que** l'on met en oeuvre le HPC en une quantité allant de 45 à 90% en poids.

3. Procédé de préparation d'une formulation suivant la revendication 1, **caractérisé en ce que** l'on met en oeuvre un HPC avec un poids moléculaire allant de 350 000 à 1 150 000.

4. Procédé de préparation d'une formulation suivant la revendication 1, **caractérisé en ce que** les particules possèdent un diamètre maximal allant de 0,5 à 2 mm.

5. Procédé de préparation d'une formulation suivant la revendication 1, **caractérisé en ce que** l'on prépare les particules par extrusion à l'état fondu et granulation.

6. Procédé de préparation d'une formulation suivant la revendication 1, **caractérisé en ce que** l'on enduit en outre les particules, d'une laque avant le remplissage des capsules.

7. Procédé de préparation d'une formulation suivant la revendication 1, **caractérisé en ce que** l'on utilise la nifédipine comme agent actif.

8. Utilisation des particules suivant la revendication 1, pour la préparation d'agents pharmaceutiques finis sous la forme de capsules.

9. Formulation à dose retard de type multiple unit avec libération contrôlée en fonction de l'agitation, administrable oralement, obtenable suivant la revendication 1.

10. Formulation à dose retard de type multiple unit avec libération contrôlée en fonction de l'agitation, administrable oralement, sous la forme de capsules, contenant de petites particules en un dosage actif, avec un diamètre allant de 0,2 à 3,0 mm, consistant en un mélange de polymère hydrophile HPC ayant un poids moléculaire moyen allant de 250 000 à 1 200 000, en une quantité allant de 40 à 95% en poids, sur base du mélange agent actif-polymère, et un taux de substitution molaire d'au moins 3, et un agent actif pharmaceutique.
